# FASCICULE DE BREVET EUROPEEN

(11) **EP 2 213 283 B1**
(45) Date de publication et mention de la délivrance du brevet: **19.02.2014**
(21) Numéro de dépôt: 09178685.5
(22) Date de dépôt: 10.12.2009
(51) Int. Cl.: A61K 8/14, A61Q 19/00, A61K 9/50, A61K 9/127

(54) **Procédé de préparation de vésicules à caractère hydrophobe comprenant une cavité interne hydrophobe**
Herstellungsverfahren für hydrophobe Vesikel einen hydrophoben Hohlraum im Innern enthaltend
Procedure for preparing hydrophobe vesicles containing an internal hydrophobe cavity

(30) Priorité: 10.12.2008 FR 0858428
(43) Date de publication de la demande: 04.08.2010
(73) Titulaire: St. Hubert, 94150 Rungis (FR)
(72) Inventeur: Dubois, Virginie, 59160, LOMME (FR); El Mafadi Jian, Samira, 44240, LA CHAPPELLE SUR ERDRE (FR); Costard, Magali, 44100, NANTES (FR); Poncelet, Denis, 44240, SUCE SUR ERDRE (FR); Picot, Arnaud, 44260, SAVENAY (FR); Marchal, Norbert, 54122, FLIN (FR); Breton, Sylvie, 78000, VERSAILLES (FR); Verdellet, Pierre, 75016, PARIS (FR)
(74) Mandataire: Colombet, Alain André

(56) Documents cités:
- EP-A- 1 116 516
- WO-A-01/05409
- WO-A-95/03787
- US-A- 5 104 736
- US-A1- 2006 002 903

## Description

La présente invention concerne un procédé de préparation de vésicules à caractère hydrophobe incorporables dans une phase hydrophobe comprenant une cavité interne hydrophobe et au moins une bicouche constituée de deux monocouches de lipides amphiphiles déterminant entre-elles un espace interstitiel hydrophile.

Dans certaines industries comme l'industrie agro-alimentaire, cosmétique, pharmaceutique, l'introduction de composés d'intérêt actifs, hydrophobes ou hydrophiles, dans des phases hydrophobes, peut se révéler difficile lorsque lesdits composés d'intérêt sont fragiles, instables, sensibles à l'oxydation.

L'encapsulation de composés d'intérêt est décrite dans la littérature, notamment au moyen de micelles ou de liposomes.

Les micelles correspondent à des gouttelettes de milieu hydrophobe en suspension dans un milieu hydrophile, délimitées par une seule monocouche de lipide amphiphile. Les parties hydrophiles des lipides amphiphiles étant orientées vers l'extérieur, et les parties hydrophobes des lipides amphiphiles sont orientées vers l'intérieur.

De la même manière, les micelles inverses correspondent à des gouttelettes de milieu hydrophile en suspension dans un milieu hydrophobe, délimitées par une seule monocouche de lipide amphiphile. Les parties hydrophobes des lipides amphiphiles étant orientées vers l'extérieur, et les parties hydrophiles des lipides amphiphiles sont orientées vers l'intérieur.

Les liposomes comportent au moins une bicouche constituée de deux monocouches de lipides amphiphiles déterminant entre-elles un espace interstitiel hydrophobe. Les parties hydrophobes des lipides amphiphiles sont orientées têtes à têtes vers l'intérieur de la bicouche, et les parties hydrophiles des lipides amphiphiles sont orientées vers l'extérieur de la bicouche. La cavité interne des liposomes est hydrophile. La monocouche externe des liposomes est hydrophile, et par conséquent les liposomes ont un caractère hydrophile, et sont incorporables dans des phases hydrophiles.

Les micelles et les liposomes sont donc des vésicules à caractère hydrophile, incorporables dans des milieux hydrophiles.

Le procédé de préparation de la présente invention fournit des vésicules à caractère hydrophobe, incorporables dans des phases hydrophobes.

Dans la littérature, on peut notamment citer l'article de Kunieda et la demande de brevet EP 1 905 425, qui décrivent des vésicules à caractère hydrophobe.

Plus précisément, Kunieda (« Formation of reversed vesicles » de Hironobu Kunieda et al., Journal of the American Chemical Society 113(3), 1051-1052, 1991) décrit des vésicules constituées d'une cavité interne hydrophobe contenant du dodécane et de bicouches constituées de deux monocouches d'émulsifiant (dodécyléther de tétraéthylène glycol). Les parties hydrophiles de l'émulsifiant sont orientées têtes à têtes vers l'intérieur de la bicouche et les monocouches comportent entre-elles un espace interstitiel rempli d'eau. Les parties hydrophobes de l'émulsifiant sont orientées vers l'extérieur de la bicouche et du dodécane est présent entre deux bicouches. Les vésicules de Kunieda qui sont concentriques ou multicouches sont incorporables dans du dodécane, qui est un milieu hydrophobe. Ce solvant est un solvant organique nocif et qui empêche toute utilisation de ces vésicules dans le domaine de l'alimentaire. Kunieda enseigne également que les vésicules ne sont pas stables et que le milieu revient sous forme de phase cristalline liquide lamellaire en l'espace de quelques heures ou jours.

La demande EP 1 905 425 décrit des vésicules à caractère hydrophobe incorporables dans une phase hydrophobe, dont la cavité interne est hydrophobe. Elles comportent au moins une bicouche constituée de deux monocouches de lipides amphiphiles déterminant entre elles un espace interstitiel hydrophile. Des vésicules concentriques ou multicouches sont décrites mais également des vésicules comprenant d'autres vésicules juxtaposées que l'on définira comme matricielles. Les parties hydrophiles des lipides amphiphiles sont orientées têtes à têtes vers l'intérieur de la bicouche, et les parties hydrophobes des lipides amphiphiles sont orientées vers l'extérieur de la bicouche.

US5104736, WO9503787, EP1116516, US2006002903 et WO0105409 se révèlent une procédé de préparation de vésicules à caractère hydrophobe et à une bicouche.

Les vésicules obtenues par le procédé selon la présente invention sont également des vésicules à caractère hydrophobe incorporables dans une phase hydrophobe, comme les huiles ou les émulsions, eau dans huile (margarine, beurre...), huile dans eau ou émulsion multiple. Leur cavité interne est hydrophobe. Elles comportent au moins une bicouche constituée de deux monocouches de lipides amphiphiles déterminant entre-elles un espace interstitiel hydrophile. Les parties hydrophiles des lipides amphiphiles sont orientées têtes à têtes vers l'intérieur de la bicouche, et les parties hydrophobes des lipides amphiphiles sont orientées vers l'extérieur de la bicouche. En particulier, l'orientation des lipides amphiphiles dans les vésicules obtenues par le procédé selon l'invention est inversée par rapport à l'orientation des lipides amphiphiles dans des liposomes. De préférence le nombre de bicouches est supérieur ou égal à deux, les bicouches sont juxtaposées ou matricielles.

La présente invention consiste en un procédé de préparation desdites vésicules. De nombreux procédés de préparation de vésicules à caractère hydrophile sont décrits dans l'art antérieur. Par exemple, le procédé pour préparer des liposomes consiste à évaporer un solvant organique dans lequel sont dissous des lipides, puis à les remettre en suspension dans un solvant aqueux (Bangham *et al.,* 1965). Cette opération doit se dérouler dans des conditions de température dépendant de la nature du (des) lipide(s) choisi(s). En effet, les liposomes ne se forment qu'à une température supérieure à celle de leur transition de phase. Une agitation est souvent nécessaire pour améliorer les résultats. Dans un milieu aqueux, le film lipidique s'hydrate et les phospholipides s'associent de manière à ne pas exposer leurs chaînes acyles au solvant : il en résulte la formation de bicouches, qui se referment en emprisonnant du solvant. Des bicouches peuvent enfermer d'autres bicouches de plus petite taille. Ainsi, lors de cette préparation, des liposomes multilamellaires se constituent en bicouches lipidiques concentriques et séparées, les unes des autres, par des couches d'eau (MLV). Ces liposomes ont des tailles très hétérogènes et possèdent un volume aqueux interne relativement faible par rapport au poids du lipide.

La présente invention consiste en un procédé de préparation de vésicules à caractère hydrophobe.

Des procédés de préparation de vésicules à caractère hydrophobe sont décrits dans les documents susmentionnés.

Ainsi, Kunieda (« Formation of reversed vesicles » de Hironobu Kunieda et al., Journal of the American Chemical Society 113(3), 1051-1052, 1991) décrit un procédé de préparation de vésicules inversées qui sont concentriques ou multicouches comprenant les étapes suivantes:
- dissolution de surfactant (dodécyléther de tétraéthylène glycol) dans du dodécane,
- addition d'eau à la solution obtenue pour former des cristaux liquides (phase cristalline liquide lamellaire),
- ajout de dodécane en excès par rapport aux cristaux liquides,
- mélange à la main des cristaux liquides et du dodécane.

La demande de brevet EP 1 905 425 décrit un procédé de préparation de vésicules comprenant les étapes de :
étape A : étalement de lipides amphiphiles,
étape B : addition d'une phase hydrophile dont sera constitué l'espace interstitiel hydrophile,
étape C : pose pour obtenir des bicouches lipidiques,
étape D : élimination de l'excès de phase hydrophile pour obtenir des films phospholipidiques hydratés,
étape E : addition d'une phase hydrophobe dont sera constitué la cavité interne hydrophobe,
étape F : agitation forte,
éventuellement étape G : décantation et récupération des vésicules.

La présente invention consiste en un procédé alternatif de préparation de vésicules à caractère hydrophobe incorporables dans une phase hydrophobe comprenant une cavité interne hydrophobe et au moins une bicouche constituée de deux monocouches de lipides amphiphiles déterminant entre-elles un espace interstitiel hydrophile qui a l'avantage d'être simple, peu coûteux, réalisé en peu d'étapes et facilement industrialisable. De préférence, lesdites vésicules sont juxtaposées ou matricielles.

Les vésicules à caractère hydrophobe obtenues par le procédé selon l'invention présentent l'avantage d'être stables, c'est-à-dire qu'elles ne coalescent pas pour former des cristaux liquides lamellaires, pendant une période d'au moins 6 semaines après leur formation, notamment au moins 2 mois, de préférence au moins 6 mois.

La présente invention concerne un procédé de préparation de vésicules à caractère hydrophobe incorporables dans une phase hydrophobe comprenant une cavité interne hydrophobe et au moins une bicouche constituée de deux monocouches de lipides amphiphiles déterminant entre elles un espace interstitiel hydrophile comprenant les étapes de :
a) fournir un milieu hydrophile, un milieu hydrophobe et des lipides amphiphiles,
b) mélanger le milieu hydrophile, le milieu hydrophobe et les lipides amphiphiles pour former les vésicules,
c) récupérer les vésicules.

Par « milieu hydrophobe », on désigne un milieu soluble dans les phases hydrophobes et insoluble dans l'eau.

Par « milieu hydrophile », on désigne un milieu soluble dans l'eau. L'eau et les solutions aqueuses sont les milieux hydrophiles préférés.

Par « lipide amphiphile », on désigne un lipide qui possède au moins un groupe hydrophile et au moins un groupe hydrophobe, par exemple des acides gras, des triglycérides, des phosphoglycérides, des phospholipides, des glycolipides, des sphingolipides.

Dans un mode de réalisation préférentiel, les vésicules sont matricielles.

Dans un mode de réalisation, une étape supplémentaire est ajoutée après l'étape c), et le procédé comprend une étape d) d'élimination du milieu hydrophobe non encapsulé dans les vésicules.

Lors de la formation des vésicules (étape b), une partie du milieu hydrophobe est encapsulée dans les vésicules (dans la cavité interne, et éventuellement entre les bicouches de lipides amphiphiles lorsque les vésicules contiennent au moins deux bicouches), et une partie du milieu hydrophobe n'est pas encapsulée et est à l'extérieur des vésicules. C'est ce milieu hydrophobe non encapsulé « en excès » qui est éliminé lors de l'étape d). Il peut éventuellement être recyclé.

De préférence, dans le procédé selon l'invention, dans les étapes a) et b), le rapport massique entre le milieu hydrophile et les lipides amphiphiles est compris entre 1/20 et 1/4, de préférence compris entre 1,5/10 et 2/10 et est de préférence de 15/85 environ, ce rapport représente le taux d'hydratation des lipides amphiphiles.
De préférence, le rapport massique entre les lipides amphiphiles et le milieu hydrophobe est compris entre 1/1 et 1/10, et est de préférence 1/3 environ. Le rapport massique entre les lipides amphiphiles et le milieu hydrophobe permet avantageusement de faire varier la taille des vésicules : la taille des vésicules est plus grande lorsque la quantité de milieu hydrophobe incorporée est faible, donc lorsque le ratio est plus grand.

Ces rapports massiques sont également particulièrement adaptés pour obtenir les vésicules recherchées, pour diminuer la quantité de milieu hydrophobe non encapsulé résiduel lors de l'étape d), et donc la durée de l'étape d).

De préférence, dans le procédé selon l'invention, le mélange lors de l'étape b) est réalisé au moyen d'un mélangeur dynamique, au moyen d'un mélangeur statique ou par sonication.

Par « un mélangeur dynamique », on entend un mélangeur comportant des pièces mobiles en rotation (rotor) et des parties statiques (stator), notamment un mélangeur rotor stator.

Par « mélangeur statique », on entend un mélangeur constitué d'éléments fixes insérés dans un tube qui se répètent de façon périodique. Le mélangeur statique ne comporte pas de pièces mobiles. L'accroissement de perte de charge engendré par les déflecteurs qui le constituent fournit l'énergie nécessaire au mélange.

Par « sonication », on entend l'utilisation d'ultra-sons à une fréquence donnée et une amplitude sur un outil de sonication appelé sonotrode.

De préférence, dans le procédé selon l'invention, le mélange lors de l'étape b) est réalisé au moyen d'un mélangeur dynamique. Le mélange au moyen d'un mélangeur dynamique est la méthode la plus efficace pour former les vésicules. Il est plus facile à mettre en oeuvre, car il ne nécessite pas de pré-mélange et les volumes de production ne sont pas limités.

Dans un mode de réalisation préféré du procédé selon l'invention, le mélange lors de l'étape b) est réalisé au moyen d'un mélangeur dynamique de type rotor/stator.

De préférence, le mélangeur dynamique est un mélangeur de type ultra-turrax couplé à une chambre de mélange.

Notamment, le mélangeur est mélangeur de type rotor/stator avec une fente de 8 mm.

Les mélangeurs dynamiques sont par exemple des mélangeurs ULTRATURRAX® ou ULTRATURRAX de type Ystral.

Dans un mode de réalisation du procédé selon l'invention, le mélange lors de l'étape b) est réalisé à une vitesse d'agitation comprise entre 3000 et 16000 rpm, notamment entre 7000 et 11000, de préférence 9000 rpm

« rpm » signifie tour par minute.

Ces gammes de vitesse d'agitation sont particulièrement adaptées pour former les vésicules.

| | |
|---|---|
| Volume de l'échantillon | Gamme de vitesse |
| <100g | 3000 - 7000 rpm |
| Entre 100 et 500 g | 7000 - 15000 rpm |
| >500 g | >15000 rpm |

Dans un mode de réalisation du procédé selon l'invention, le mélange lors de l'étape b) dure entre 1 et 10 minutes, notamment entre 2 et 7 minutes, de préférence 5 minutes.

Ces durées de mélange sont les durées optimales pour la formation de vésicules.

Dans un mode de réalisation du procédé selon l'invention, l'étape c) est réalisée par centrifugation ou par ultrafiltration tangentielle.

Par « centrifugation », on désigne la technique utilisant la force centrifuge pour séparer les vésicules du milieu hydrophobe non encapsulé résiduel.

Par « ultrafiltration tangentielle », on désigne une filtration par membrane, notamment une membrane organique poreuse, par laquelle on force le mélange issu de l'étape b) à passer à travers une membrane semiperméable qui retient les vésicules et laisse passer le milieu hydrophobe non encapsulé résiduel.

L'étape c) permet de récupérer les vésicules formées par le procédé selon l'invention en les séparant du milieu hydrophobe non encapsulé résiduel, qui peut éventuellement être recyclé.

De préférence, l'étape c) est réalisée par centrifugation.

Dans un mode de réalisation préféré du procédé selon l'invention, l'étape c) est réalisée par centrifugation à une vitesse comprise entre 200 et 1000 G, notamment entre 400 et 800 G, de préférence 600 G.

Ces gammes de vitesse sont particulièrement adaptées pour récupérer les vésicules formées.

Dans un mode de réalisation préféré du procédé selon l'invention, l'étape c) est réalisée par centrifugation et dure 5 à 30 minutes, notamment de 7 à 15 minutes, de préférence 10 minutes.

Ces durées de centrifugation sont suffisantes pour isoler les vésicules du milieu hydrophobe non encapsulé résiduel et pour récupérer les vésicules formées.

Dans un mode de réalisation du procédé selon l'invention, l'étape d) est réalisée par lavage avec une phase hydrophobe différente du milieu hydrophobe utilisé lors des étapes a), b) et c).

Dans un mode de réalisation préféré du procédé selon l'invention, le milieu hydrophobe comprend une huile choisie parmi les huiles neutres, les huiles essentielles, les huiles polyinsaturées marines ou les huiles polyinsaturées végétales.

Par « huile neutre », on désigne une huile notamment choisie parmi les huiles d'arachide (*Arachis hypogea*), d'adjouaba (*Haematostaphis barten),* d'avocat (*Persea americana*), de babassu (*Orbignya oleifera*), de beurre de karité (*Vitellaria paradoxa*), de beurre de cacao (*Theobroma cacao*), de chufa (*Cyperus esculentus*), de crambe (*Crambe abyssinica*), de cuphea (*Cuphea tolucana, Cuphea wrightii, Cuphea laminuligena, Cuphea ilavea* ou *Cuphea leptopoda*), d'érable (*Acer saccharum* ou *Acer saccharinum*), de Gokhru (*Tribulus terrestris*), de noisette (*Corylus avellana*), de noix de coco (*Cocus nucifera*), de noix de Macadamia (*Macadamia tetraphylla*), de noix du Cambodge (*Irvingia malayana*), de noix pili (*Canarium ovatum*), de noyau d'abricot (*Prunus armeniaca*), d'olive (*Olea europaea*), de palme (*Elaeis guineensis*), de palmiste (*Elaeis guineensis*), de pépins de mangue (*Mangifer indica*), de pistache (*Pistacia atlantica*)..., et des fractions de ces huiles (comme par exemple les oléines et stéarines de palme ou de palmiste). Ces huiles neutres peuvent être utilisées seules ou en mélange.

Par « huile essentielle », on désigne le liquide concentré et hydrophobe des composés aromatiques volatiles d'une plante, par exemple les huiles de citron, de lavande, d'eucalyptus, de camphre, de sauge, de thym, de girofle...

Par « huiles polyinsaturées marines », on désigne notamment les huiles de poisson, de crustacé ou d'algues.

Par « huiles polyinsaturées végétales », on désigne les huiles notamment choisies parmi :
- les huiles polyinsaturées végétales riches en acides gras oméga 3, comme les huiles d'amarante (*Amaranthus viridis*), d'argousier (*Hippophae rhamnoides*), de bélize (*Ximenia americana*), de cameline (*Camelina sativa*), de chanvre (*Cannabis sativa*), de chia (*Salvia hispanica*), de colza (*Brassica napus*), d'échium (*Echium* sp.), de fenugrec (*Trigonella foenum-graecum*), de lin (*Linum usitatissimum*), de matthiole (*Matthiola tricuspidata*), de moutarde blanche *(Brassica alba*), de noix (*Juglans regia*), de pépin de canneberge (*Vaccinium oxycoccos*), de pépin de cassis (*Ribes nigrum*), de pépin de framboise (*Rubus idaeus*), de perilla (*Perilla frutescens*), de pourpier (*Portulaca oleracea*), de salicorne (*Salicornia europaea*)
- les huiles polyinsaturées végétales riches en acides gras oméga 6 comme les huiles d'amarante (*Amaranthus caudatus ; Amaranthus cruentus* ou *Amaranthus hypocondriacus*), d'argan (*Argania spinosa*), d'artichaut (*Cynara scolymus*), d'avoine (*Avena sativa*), de bourrache (*Borago officinalis*), de cactus *(Opuntia ficus indica*), de carthame (*Carthamus tintorius*), de cumin (*Cuminum cymimum*), de cumin noir (*Nigella sativa*), de germe de blé (*Triticum aestivum*)*,* de graine de coton (*Gossypium hirsutum*), de graine de paprika (*Capsicum annuum*), de graine de tabac (*Nicotiana tabacum*), d'haricot africain (*Pentaclethra macrophylla),* de kénaf (*Hibiscus cannabinus*), de lupin (*Lupinus mexicanus*), de maïs (*Zea mais*), de Niger (*Guizotia abyssinica*)*,* de noyer cendré (*Juglans cinerea*), d'oignon (*Allium cepa*), d'onagre (*Oenothera biennis*), de pépin de citrouille (*Cucurbita pepo*), de pépin de melon (*Cucumis melo*), de pépin de pastèque (*Citrullus* sp.), de pépin de raisin (*Vitis vinifera*), de quinoa (*Chenopodium quinua*), de ratanjyot (*Jatropha curcas*), de salicorne (*Salicornia bigelovii*)*,* de sarrasin (*Polygonum fagopyrum*), de sésame (*Sesamum indicum*), de soja (*Glycine max*), de son de riz (*Oryza sativa*), de tournesol (*Helianthus annuus*) *...*

De plus, les vésicules comprenant de l'huile, des lipides amphiphiles et un milieu hydrophile obtenues par le procédé selon l'invention sont stables, et ne coalescent pas pour former des cristaux liquides lamellaires, pendant une période d'au moins 6 semaines après leur formation.

Dans un mode de réalisation du procédé selon l'invention, le milieu hydrophobe est une huile polyinsaturée marine choisie parmi les huiles de poissons.

Dans un mode de réalisation préféré du procédé selon l'invention, les lipides amphiphiles sont des phospholipides.

Par « phospholipides », on désigne les lipides possédant un groupe phosphate, soit les phosphoacylglycérols (plus connus sous le nom de glycérophospholipides), les phosphosphingolipides et les phosphonosphingolipides; et les phosphosaccharolipides.

Dans un mode de réalisation du procédé selon l'invention, les lipides amphiphiles sont des lécithines, de préférence lécithine de soja.

Dans un mode de réalisation particulier, les lipides amphiphiles peuvent être stabilisés par au moins un composé stabilisateur. Il peut s'agir par exemple d'un composé de type tocophérol comme l'alpha-tocophérol, ou de type stérol comme le cholestérol.

Dans un mode de réalisation du procédé selon l'invention, le milieu hydrophile est une phase aqueuse.

Dans un mode de réalisation du procédé selon l'invention, la phase aqueuse comprend au moins un émulsifiant.

Par « émulsifiant », on désigne des stabilisateurs d'émulsion.

Les inventeurs ont démontré que pour former des vésicules, un écart entre la balance hydrophile/lipophile (HLB) du milieu hydrophobe et celle des lipides amphiphiles est nécessaire. Or, les milieux hydrophobes ont un caractère hydrophobe plus ou moins important, qui est par exemple lié à la longueur de la chaîne des acides gras, au nombre d'insaturations dans les chaînes carbonées ... Lorsque le milieu hydrophobe est très hydrophobe, une dissolution des lipides amphiphiles dans le milieu hydrophobe est observée, ce qui empêche la formation des bicouches lipidiques nécessaires à la formation des vésicules.

Pour défavoriser cette dissolution, lorsque le milieu hydrophobe est très hydrophobe, un émulsifiant de HLB plus élevée que celle du milieu hydrophobe est ajouté à la phase aqueuse, ce qui permet d'augmenter fictivement l'écart entre l'hydrophobicité du milieu hydrophobe et celle des lipides amphiphiles.

Dans un mode de réalisation du procédé selon l'invention, le au moins un émulsifiant est du maléate de sorbitane polyoxyethyléné (Tween 20^{®}), du sorbitane triooléate (Span 85^{®}) ou du dodécylsulfate de sodium (SDS).

Dans un mode de réalisation du procédé selon l'invention, la phase aqueuse comprend au moins un agent texturant.

Par « agent texturant », on désigne un composé chimique qui favorise la formation des vésicules et améliore leur stabilité. L'ajout d'agent texturant permet notamment d'obtenir des vésicules ayant des contours plus nets (le contour étant représentatif de la bicouche la plus externe de la vésicule), c'est-à-dire pour lesquelles les lipides amphiphiles diffusent moins et restent dans la bicouche lipidique.

Dans un mode de réalisation du procédé selon l'invention, au moins un agent texturant est choisi parmi la gomme arabique, le chitosan et le caséinate de sodium.

Par exemple, une solution aqueuse de gomme arabique à 15%, une solution aqueuse de chitosan à 1% ou une solution aqueuse de caséinate de sodium à 1,5% sont particulièrement adaptées pour mettre en oeuvre le procédé selon l'invention.

Dans un mode de réalisation du procédé selon l'invention, la phase aqueuse comprend au moins un agent antioxydant.

Par « agent antioxydant », on désigne un composé chimique qui diminue ou empêche l'oxydation des autres substances chimiques du milieu.

Dans un mode de réalisation du procédé selon l'invention, au moins un antioxydant est l'acide éthylène-diamine-tétraacétique (EDTA).

Dans un mode de réalisation particulier, dans le procédé selon l'invention, le milieu hydrophile comprend au moins un agent faisant barrière à l'oxygène. Parmi les agents faisant barrière à l'oxygène, on peut citer les gels d'hydrocolloïdes peu hydratés, la cire d'abeille, de l'isolat de protéines de lactosérum, de l'isolat de protéines de soja, de la β-lactoglobuline, de la caséine, des sels de caséine comme le caséinate de sodium, du sorbitol, du glycérol, utilisés seuls ou en mélange.

Dans un autre mode de réalisation, dans le procédé selon l'invention, le milieu hydrophile comprend au moins un agent capable d'augmenter la rigidité de la bicouche. Ceci permet d'augmenter la stabilité des opposomes lors des procédés de séparation (centrifugation, ultrafiltration...etc). Cet agent peut être choisi parmi les hydrocolloïdes. On peut citer, comme exemple d'hydrocolloïdes, l'amidon natif (de blé, de riz, de maïs, de paprika, de pomme de terre...), l'amidon réticulé, l'amidon prégélatinisé, l'amylopectine ou alors les produits de dégradation de l'amidon comme les maltodextrines de différents degrés d'hydrolyse, ou encore des gommes. Des exemples de gommes sont les gommes d'acacia, les gommes guar, les gommes xanthane, les gommes adragante, les gommes karaya, les gommes tara, les gommes gellane, le sorbitol, le sirop de sorbitol, le mannitol, le glycérol, le glucomannane, le stéarate de polyoxyéthylène (8), le stéarate de polyoxyéthylène (40), le monolaurate de polyoxyéthylène sorbitane (polysorbate 20), le monooléate de polyoxyéthylène de sorbitane (polysorbate 80), le monopalmitate de polyoxyéthylène sorbitane (polysorbate 40), le monostéarate de polyoxyéthylène sorbitane (polysorbate 60), le tristéarate de polyoxyéthylène sorbitane (polysorbate 65), la pectine, la pectine amidée, les phosphatides d'ammonium, l'acétate isobutyrate de saccharose, les esters glycériques de résine de bois, l'acide alginique, les alginates de sodium, de potassium, d'ammonium, de calcium ou de propane-1,2-diol, l'agar-agar, les carraghénanes, les algues euchema transformées ou la farine de graines de caroube, utilisés seuls ou en mélange.

Dans un mode de réalisation du procédé selon l'invention, la phase hydrophobe de l'étape d) est une huile différente du milieu hydrophobe utilisé lors des étapes a), b) et c).

Dans un mode de réalisation du procédé selon l'invention, la phase hydrophobe de l'étape d) est de l'huile de colza.

Par exemple, lorsque le milieu hydrophobe est de l'huile de poisson, l'utilisation d'huile de colza lors de l'étape d) est particulièrement adaptée.

Dans un mode de réalisation, le milieu hydrophile comprend au moins un composé d'intérêt hydrophile et/ou le milieu hydrophobe comprend au moins un composé d'intérêt hydrophobe. Les composés d'intérêt sont notamment des substances actives tels que par exemples des principes actifs ou ingrédients pharmaceutiques et/ou cosmétiques, ou encore des principes actifs ou des ingrédients alimentaires.

Parmi les composés d'intérêt hydrophobes, on peut citer :
- les principes actifs ou ingrédients pharmaceutiques et/ou cosmétiques hydrophobes pouvant être choisis parmi le paracétamol, le pindolol, le probucol,
- les principes actifs ou ingrédients alimentaires hydrophobes pouvant être choisis parmi les antioxydants (extrait de romarin, de thé vert, mélange de tocophérols), les acides gras libres (pouvant être choisis parmi l'EPA, le DHA, l'acide arachidonique... ou des acides gras essentiels comme l'acide linoléique, l'acide alpha-linolénique), les stérols végétaux, les stanols végétaux, les polycosanols, les vitamines liposolubles (A, D, E, K), les colorants (caroténoïdes, flavonoïdes...), les composés aromatiques (diacétyle...).

Parmi les composés d'intérêt hydrophiles, on peut citer :
- les principes actifs ou ingrédients pharmaceutiques et/ou cosmétiques hydrophiles pouvant être choisis parmi les antitumoraux (adriamycine, actimycine, mitomycine, 1 -arabinofuranosyl cytosine, cisplatine), les antiviraux (interféron), les aminoglucosides (gentamicine), les antibiotiques ( -lactam, sulbenicilline, céfotiame, cefmenoxime), les hormones peptidiques (TRH, leuprolide, insuline), les agents immunopotentialisateurs (muramyldipeptide, muramyltripeptide), les protéines (immunoglobulines, toxines), les agents anti-allergiques, antimycotiques, cytostatiques ou de diagnostic, les anxiolytiques, les contraceptifs, les sédatifs ;
- les principes actifs ou ingrédients alimentaires hydrophiles pouvant être choisis parmi les sels minéraux (calcium, chlore, magnésium, phosphore, potassium, sodium, soufre), les oligo-éléments (aluminium, brome, cuivre, cobalt, fer, fluor, manganèse, molybdène, iode, sélénium, silicium, vanadium, zinc), les glucides (comme le glucose, le galactose, le mannose, le maltose, le maltotriose), les acides aminés (alanine, arginine, asparagine, acide aspartique, cystéine, acide glutamique, glutamine, glycine, histidine, isoleucine, leucine, lysine, méthionine, phénylalanine, proline, sérine, thréonine, tryptophane, tyrosine, valine), les peptides, les protéines, les vitamines hydrosolubles, les arômes (de citron, de vanille, de fraise, de café, de noisette, de framboise...), les polyols.

L'invention est illustrée par les exemples suivants.

Les différents supports utilisés pour la formation de vésicules par la méthode indirecte sont répertoriés dans le tableau ci-dessous :

| **SUPPORT** | **CARACTERISTIQUES** |
|---|---|
| Tube en verre borosilicaté | - Col à vis |
| | - Longueur 100 mm |
| | - Diamètre 13 mm |
| | - Epaisseur 1,5 mm |
| Cylindre rotatif | - Hauteur du cylindre : 32 mm |
| | - Diamètre du cylindre : 22 mm |
| Tige en verre | - Longueur : 155 mm |
| | - Diamètre : 6 mm |
| Tige plastique | - Longueur : 25 mm |
| | - Diamètre : 10 mm |
| Roux inox | - Diamètre : 80 mm |

### Méthodes de caractérisation

La structure des vésicules (taille, homogénéité de taille, aspect général) est déterminée par observation microscopique. Une gouttelette de la suspension résultant de la production de vésicules est déposée entre lame et lamelle puis observée à l'aide d'un microscope. Trois types de microscopie ont été utilisés :
- Microscopie optique : cette technique permet de visualiser la morphologie, la taille et l'aspect granuleux des vésicules ainsi que la présence de débris de couche de lipide amphiphile. Le microscope utilisé est le modèle LEICA équipé d'un analyseur d'images Visilog.
- Microscopie à fluorescence : la lécithine de soja fluoresce naturellement à la même longueur d'onde que la fluorescéine, elle est donc facilement observable avec un microscope à fluorescence. Le microscope utilisé est le modèle BH2 RFCA de Olympus.
   La forme des vésicules notamment, la présence de vésicules de milieu hydrophobe dans les vésicules (vésicules matricielles), ainsi que la présence de débris (diffusion de la fluorescence) peuvent être observées.
- Microscopie confocale : cette microscopie permet de réaliser des images de très faible profondeur de champ (environ 400 nm) appelées « sections optiques ». En positionnant le plan focal de l'objectif à différents niveaux de profondeur dans l'échantillon, il est possible de réaliser des séries d'images à partir desquelles on peut obtenir une représentation tridimensionnelle de l'objet.

### Rendement de l'encapsulation

Le rendement de l'encapsulation est déterminé en utilisant une centrifugeuse modèle SIGMA 2K165.

Cette méthode permet d'éliminer le milieu hydrophobe non encapsulé dans le mélange et ainsi déterminer le pourcentage de vésicules et de milieu hydrophobe encapsulé dans le mélange. Les conditions de centrifugation sont les suivantes : 600 G pendant 10 minutes à 20°C.

### EXEMPLE 1 : Rapport massique entre les lipides amphiphiles et le milieu hydrophobe

Dans cet exemple, les lipides amphiphiles sont de la lécithine de soja fournie par Cargill Texturizing Solutions^{®} et le milieu hydrophobe est de l'huile de poisson (Marinol™ C-38^{®}) fournie par Lipid nutrition^{®}. Le taux d'hydratation de la lécithine est de 15% (le milieu hydrophile est de l'eau). L'étape b) de mélange est réalisée par un mélangeur dynamique de type Ultraturrax Ystral X40^{®} (fente 8 mm) à 16 000 rpm pendant 5 minutes.

Des ratios lipide amphiphile / milieu hydrophobe de 1/3, 1/5 et 1/10 ont été testés.

| **Ratio lipide amphiphile/milieu hydrophobe** | **Taille des vésicules** | **Taux d'encapsulation** |
|---|---|---|
| 1/3 | 10-50 µm | 40 % |
| 1/5 | 5-30 µm | 42 % |
| 1/10 | 5-20 µm | 44 % |

Le taux d'encapsulation correspond au pourcentage de milieu hydrophobe pour 100 grammes de vésicules et n'est pas significativement différent avec les trois ratios comparés.

La taille des vésicules est plus grande lorsque la quantité de milieu hydrophobe incorporée est faible (ratio 1/3).

D'après ces résultats, la taille des vésicules ne semble pas influencer la capacité des vésicules à contenir du milieu hydrophobe.

Toutefois, le ratio 1/3 présente l'avantage de réduire le temps d'élimination du milieu hydrophobe non encapsulé dans les vésicules lors de l'étape d), de diminuer la quantité de milieu hydrophobe à recycler et ainsi la durée de l'étape d).

### EXEMPLE 2 : Ratio entre le milieu hydrophile et les lipides amphiphiles

L'effet sur la formation des vésicules lié au pourcentage d'hydratation de la lécithine (Pₑₐᵤ/P_{lécithine}) a été évalué par observations microscopiques.

La quantité d'eau est ajoutée en fonction du taux d'hydratation de la lécithine voulu.

Plusieurs pourcentages d'hydratation, allant de 10 à 30% (p/p) de la lécithine ont été testés.

L'homogénéisation a été réalisée à l'aide de l'ultra turrax Yellow Line^{®} (vitesse d'agitation de 7400 rpm pendant 2 minutes). Le ratio lipide amphiphile (lécithine de soja fournie par Cargil Texturizing Solutions^{®}) / milieu hydrophobe (huile de poisson Marinol™ C-38^{®}) est de 1/10, pour des quantités totales mises en jeu de 11,4 grammes. Les observations réalisées pour chaque taux d'hydratation testé sont regroupées dans le tableau :

| **Taux d'hydratation de la lécithine de soja** | **Aspect des vésicules*** | **Observations*** |
|---|---|---|
| 10% | Vésicules sphériques | Les vésicules ont tendance à s'agglomérer |
| 15% | Vésicules sphériques et ovoïdes | Peu d'amas |
| 20% | Vésicules sphériques et ovoïdes | Peu d'amas |
| 25% | Vésicules sphériques, ovoïdes et multiformes | Quelques débris de lécithine de soja Beaucoup d'amas |
| 30% | Vésicules sphériques, ovoïdes et multiformes | Beaucoup de débris de lécithine de soja Agglomérats |

| | | |
|---|---|---|
| ^{*}Les vésicules sont observées par microscopie classique et à fluorescence | | |

Les essais les plus concluants sont ceux réalisés à un taux d'hydratation de la lécithine de 15 et 20%.

### EXEMPLE 3 : Ajout d'additif (agent texturant ou antioxydant) dans le milieu hydrophile.

Dans cet exemple, les lipides amphiphiles sont de la lécithine de soja (fournie par Cargil Texturizing Solutions^{®}) et le milieu hydrophobe est de l'huile de poisson (Marinol™ C-38^{®}). Le taux d'hydratation de la lécithine est de 15%. L'étape b) de mélange est réalisée par un mélangeur dynamique de type Ultraturrax Ystral X10^{®} outil dispersant 25DG à 10 000 rpm pendant 30 secondes puis 7600 rpm pendant 90 secondes. Le ratio lipide amphiphile / milieu hydrophobe est de 1/5.

Le milieu hydrophile est soit :
- de l'eau,
- une solution aqueuse de chitosan à 1 % (agent texturant),
- une solution aqueuse de EDTA à 2 % (agent anti-oxydant),
- une solution aqueuse de gomme arabique à 15 % (agent texturant).

Des essais ont permis de sélectionner les concentrations optimales susmentionnées de chaque additif.

Les milieux obtenus à chaque essai ont été observés par fluorescence, et centrifugés pour déterminer le taux d'encapsulation.

| Milieu hydrophile | Microscopie classique et à fluorescence | Taux d'encapsulation (%) |
|---|---|---|
| eau | Contours des vésicules +/- nets Vésicules de petites tailles et multiformes Vésicules granuleuses Diffusion de la lécithine Quelques débris | 38 |
| solution aqueuse de chitosan à 1 % | Contours des vésicules +/- nets Vésicules plus grosses et multiformes Vésicules granuleuses Moins de diffusion de la lécithine Pas de débris | 38 |
| solution aqueuse de EDTA à 2% | Contours des vésicules plus nets Vésicules plus grosses, sphériques Vésicules granuleuses Peu de diffusion de la lécithine Pas de débris | 37 |
| solution aqueuse de gomme arabique à 15% | Contours des vésicules plus nets Vésicules plus grosses, sphériques Vésicules granuleuses Peu de diffusion de la lécithine Pas de débris | 37 |

D'une manière générale, l'utilisation d'agent texturant ou antioxydant n'a pas d'effet significatif sur le taux d'encapsulation.

On observe toutefois des vésicules de forme plus régulière et de contour bien délimité lorsque le milieu hydrophile comporte de l'EDTA ou de la gomme arabique.

### EXEMPLE 4 : Ajout d'émulsifiant dans le milieu hydrophile

Trois tensioactifs de HLB différents sont utilisés : le Span 85, le Tween 20 et le SDS. Leur HLB est respectivement 1,8, 16,7 et 40,0.

Les conditions expérimentales appliquées sont les suivantes :
Milieu hydrophobe : huile de poisson Marinol™ C-38^{®} ou huile de colza (fournie par St Hubert^{®})
lipide amphiphile : lécithine de soja fournie par Cargil Texturizing Soultions^{®}
Taux d'hydratation de la lécithine = 15%
Quantité totale mise en jeu = 2,5g
Ratio Lécithine/Huile de poisson =1/5
Homogénéisation = 2500 tr/mn pendant 120 sec (mélangeur) (vortex).

Le tableau ci-après décrits les essais réalisés, et les observations qui ont suivi :

| **Tensio-actif** | **HLB** | **Huile** | **Observations** |
|---|---|---|---|
| Span 85 | 1,8 | Huile de poisson Marinol C38 | Formation de vésicules et présence d'amas de lécithine |
| | | Huile de colza | Déphasage |
| Tween 20 | 16,7 | Huile de poisson Marinol C38 | Formation de vésicules multiforme et présence d'amas de lécithine |
| | | Huile de colza | Formation de vésicules |
| SDS | 40 | Huile de colza | Formation de vésicules |

En ajoutant un émulsifiant très lipophile (Span 85), la formation des vésicules avec l'huile de poisson est possible, mais pas avec de l'huile de colza.

En ajoutant un émulsifiant hydrophile (Tween 20), la formation des vésicules avec l'huile de poisson est encore possible mais difficile, et devient possible avec de l'huile de colza.

En ajoutant un émulsifiant très hydrophile (SDS), la formation des vésicules avec l'huile de colza est possible, et d'autant plus lorsqu'on augmente la concentration en SDS.

La lécithine est plus soluble dans l'huile de colza que dans l'huile de poisson, ce qui la rend moins disponible à former une membrane phospholipidique. En ajoutant un émulsifiant hydrophile tel que le SDS, on augmente le caractère hydrophile de la lécithine, ce qui diminue sa solubilité dans l'huile de colza et permet ainsi la formation de membrane phospholipidique.

En effet, moins le milieu hydrophobe à encapsuler est hydrophobe (par exemple chaîne d'acides gras plus courtes, degré d'insaturation plus faible), plus l'ajout d'émulsifiant de HLB élevé est nécessaire pour modifier le caractère hydrophobe de la lécithine.

### EXEMPLE 5 : Nature du milieu hydrophobe

Des essais de formation de vésicules ont été réalisés avec trois types d'huile.

Les conditions expérimentales suivantes ont été appliquées pour tous les essais :
Quantité totale mise en jeu = 25 g
lipide amphiphile : lécithine de soja fournie par Cargil Texturizing Soultions^{®}
Taux d'hydratation de la lécithine de soja = 15%
Ratio Lécithine/Huile = 1/5
Homogénéisation = 7000 tr/mn pendant 120 sec (Ystral X10 Outil disperseur 25DG^{®})

Le tableau ci-dessous décrit les huiles testées, les observations faites sur les vésicules formées avec celles-ci, ainsi que le taux d'encapsulation obtenu après centrifugation d'un échantillon.

| **Huile** | **Caractéristiques** | **Observations** | **Taux d'encapsulation** |
|---|---|---|---|
| Huile de poisson Marinol C38^{®} (Lipid Nutrition^{®}) | EPA+DHA = 38% | vésicules granuleuses Contour bien délimité | 38% |
| Huile de poisson Marinol D40^{®} (Lipid Nutrition^{®}) | EPA+DHA = 47% | vésicules granuleuses Contour bien délimité | 37% |
| Huile à base d'algues (Martek^{®}) | EPA+DHA = 38% | Pas de formation de vésicules dans les conditions testées | - |

Des vésicules granuleuses et bien délimitées sont obtenues avec les deux huiles de poisson, alors que l'huile Martek^{®} ne permet pas la formation de vésicules ; cette huile est certainement moins hydrophobe que les Marinol C38^{®} et D40^{®}.

L'huile Martek^{®} a une concentration en DHA et EPA comparable aux huiles de poisson. En revanche, elle contient également un fort pourcentage en acides gras saturés tels que le c14 :0 (≈ 8%) et le C16 :0 (≈20%).

Le DHA (acide docosahexaénoique) et l'EPA (acide éicosapentaénoïque) sont des acides gras de la famille des Oméga 3.

### EXEMPLE 6 : Vitesse d'agitation lors du mélange et durée d'agitation

La vitesse et le temps d'homogénéisation avec l'ultra-turrax X40^{®} ont été optimisés, ainsi que le choix de l'outil dispersant pour la formation des vésicules.

Dans cet exemple, le lipide amphiphile est de la lécithine de soja fournie par Cargil Texturizing Solutions^{®} dont le taux d'hydratation est de 15%, le milieu hydrophobe est de l'huile de poisson Marinol C38^{®} (Lipid Nutrition^{®}), avec un ratio lécithine / huile de 1/5.

### a) Vitesse d'homogénéisation

### * avec l'ultra-turrax X40^{®}

Les observations réalisées au cours de ces essais figurent dans le tableau suivant :

| **Vitesse d'homogénéisation** | **Taille de l'échantillon** | **Observations** |
|---|---|---|
| 3500 tr/min | 1200g | Turbulence insuffisante Pas de formation de vésicules |
| 7000 tr/min | 1200g | Mélange de l'échantillon non homogène Formation de vésicules Amas de lécithine |
| 9000 tr/min | 1200g | Turbulence importante, mélange homogène de l'échantillon Formation de vésicules Peu d'amas de lécithine |
| 16000 tr/min | 1200g | Turbulence importante, mélange homogène de l'échantillon Formation de vésicules Peu d'amas de lécithine |

Pour homogénéiser un échantillon de 1200 g dans un bécher de 2 litres, la vitesse minimale nécessaire est de 9000 tr/min.

Une vitesse plus importante n'améliore pas la formation de vésicules, mais entraîne au contraire un échauffement plus important du mélange qui pourrait s'avérer préjudiciable quant à la stabilité des vésicules.

### * avec l'ultra Turrax Yellow Line^{®}

La vitesse d'homogénéisation a également été testée avec l'ultra Turrax Yellow Line^{®}.

La vitesse optimale correspond à 7400 tr/min avec cet équipement.

### b) Durée d'agitation

L'effet du temps d'homogénéisation sur la formation et la microstructure des vésicules a également été évalué pour une vitesse d'agitation de vitesse d'homogénéisation 9000 tr/min.

### * avec l'ultra-turrax X40^{®}

Des prélèvements de vésicules sont effectués à différents temps d'homogénéisation et observés au microscope

Les observations réalisées au cours de ces essais figurent dans le tableau suivant :

| **Temps d'homogénéisation** | **Aspect des vésicules** |
|---|---|
| 1 | Peu de vésicules Amas de lécithine |
| 2 | vésicules mieux formées Quelques amas de lécithine |
| 4 | vésicules mieux formées, sphériques et ovoïdes Quelques amas de lécithine |
| 5 | vésicules sphériques et ovoïdes |
| 6 | vésicules sphériques et ovoïdes |
| >10 | vésicules sphériques et ovoïdes |

Un temps d'homogénéisation de 5 minutes est nécessaire pour obtenir des vésicules sphériques et ovoïdes, et minimiser les amas de lécithine. Après ce temps minimal de mélange, l'aspect des vésicules est semblable, mais on observe un échauffement important du mélange.

### * avec l'ultra turrax DI 25BNasic Yellow Line^{®}

Le temps d'homogénéisation a également été testé avec l'ultra turrax DI 25BNasic Yellow Line^{®}, avec un temps optimum égal à 2 minutes.

### EXEMPLE 7 : Récupération des vésicules et élimination du milieu hydrophobe non encapsulé

Deux techniques ont été envisagées pour la récupération des vésicules (étape c) : l'ultrafiltration tangentielle et la centrifugation.

Pour l'ultrafiltration tangentielle, une membrane en polyéthylène ayant une surface membranaire de 0,01 m² a été utilisée, avec un diamètre interne de 5 mm et un diamètre de pores de 1 mm. Cette technique est facilement transposable à l'échelle industrielle, idéalement avec un système de nettoyage de la membrane en continu pour éviter la saturation de la membrane.

Les paramètres de centrifugation ont été optimisés afin d'éliminer le maximum de milieu hydrophobe non encapsulé du surnageant, sans altérer les vésicules. Une vitesse de centrifugation de 600 G pendant 10 minutes permet de répondre à ces critères.

Des essais ont été menés pour comparer ces deux techniques et comparer la structure des vésicules ayant subi une ultrafiltration ou une centrifugation.

Avant les traitements de concentration et de lavage des vésicules, leur forme est ovoïde et sphérique, elles sont granuleuses, leur contour est bien délimité. La taille des vésicules est de 10 à 50 µm. On observe peu de débris, ceci étant confirmé en microscopie à fluorescence.

Après ultrafiltration, on observe une diminution de taille des vésicules. Par microscopie à fluorescence, le contour des vésicules apparaît plutôt diffus. L'ultrafiltration fractionne les vésicules. Après centrifugation, la taille des vésicules n'a pas diminué. L'observation à la fluorescence permet de voir la présence de vésicule interne dans les vésicules. Le contour de celles-ci semble également préservé.

La technique de récupération des vésicules par ultrafiltration nécessite plusieurs passages du mélange à travers le module de filtration, à l'inverse de la centrifugation. La diminution de taille peut s'expliquer par le fait que la lécithine se solubilise dans l'huile de colza (phase hydrophobe utilisée pour le lavage lors de l'étape d)), ce qui expliquerait le contour diffus des vésicules récupérées par ultrafiltration.

Le mélange final dans l'huile de colza de chaque technique de récupération des vésicules est centrifugé afin de calculer le taux d'encapsulation.

Les taux sont sensiblement identiques quelle que soit la méthode de concentration et de lavage des vésicules (42% par ultrafiltration et 40% par centrifugation).

L'ultrafiltration fractionne les vésicules, mais le taux d'encapsulation reste similaire puisque les vésicules sont matricielles.

### EXEMPLE 8 : Stabilité des vésicules

La stabilité de quatre types de vésicules comportant des milieux hydrodrophiles différents (eau, solutions aqueuses de chitosan, d'EDTA ou de gomme arabique) a été étudiée.

Les conditions expérimentales suivantes ont été appliquées pour tous les essais :
Quantité totale mise en jeu = 150g
Milieu hydrophobe : huile de poisson Marinol™ C-38^{®}
lipide amphiphile : lécithine de soja fournie par Cargil Texturizing Solutions^{®}
Taux d'hydratation de la lécithine = 15%
Ratio Lécithine/Huile = 1 :5
Homogénéisation = 10000 tr/mn pendant 300 sec, puis 7600 tr/min pdt 90 sec
Ystral X10 Outil disperseur 25DG^{®}
Récupération et lavage des vésicules par centrifugation

Les concentrations en huile de poisson et en lécithine sont suivies au cours du temps pendant 6 semaines. On n'observe pas de variation importante des concentrations en huile de poisson et lécithine au cours des 6 semaines.

Le tableau suivant donne les variations observées des concentrations calculées.

| **Milieu hydrophile** | **Δ % huile de poisson** | **Δ %lécithine** |
|---|---|---|
| Eau | 1,15 | 0,41 |
| Chitosan(1%) | 1,35 | 0,36 |
| EDTA (2%) | 1,97 | 0,4 |
| Gomme Arabique (15%) | 1,23 | 0,28 |

Les variations de la concentration en huile de poisson ne sont pas supérieures à 2%, celle de la concentration en lécithine est inférieure à 0,5%, et ceci quelque soit le milieu hydrophile.

Les vésicules sont stables au cours du temps : les différents milieux hydrophiles testés ne changent en rien cette stabilité.

## Revendications

1. Procédé de préparation de vésicules à caractère hydrophobe incorporables dans une phase hydrophobe comprenant une cavité interne hydrophobe et au moins une bicouche constituée de deux monocouches de lipides amphiphiles déterminant entreelles un espace interstitiel hydrophile comprenant les étapes de :
a) fournir un milieu hydrophile, un milieu hydrophobe et des lipides amphiphiles,
b) mélanger le milieu hydrophile, le milieu hydrophobe et les lipides amphiphiles pour former les vésicules, le rapport massique entre le milieu hydrophile et les lipides amphiphiles étant compris entre 1/20 et 1/4.
c) récupérer les vésicules.

2. Procédé selon la revendication 1, **caractérisé en ce que** le rapport massique entre le milieu hydrophile et les lipides amphiphiles est compris entre 1,5/10 et 2/10.

3. Procédé selon l'une quelconque des revendications précédentes, **caractérisé en ce que** le mélange lors de l'étape b) est réalisé au moyen d'un mélangeur dynamique, d'un mélangeur statique ou par sonication.

4. Procédé selon la revendication 3, **caractérisé en ce que** le mélange lors de l'étape b) est réalisé par mélangeur dynamique de type rotor/stator.

5. Procédé selon l'une quelconque des revendications précédentes, **caractérisé en ce que** les lipides amphiphiles sont des phospholipides, notamment des lécithines, de préférence lécithine de soja.

6. Procédé selon l'une quelconque des revendications précédentes, **caractérisé en ce que** le milieu hydrophile est une phase aqueuse.

7. Procédé selon la revendication 6, **caractérisé en ce que** la phase aqueuse comprend au moins un émulsifiant, de préférence du maléate de sorbitane polyoxyethyléné, du sorbitane triooléate ou du dodécylsulfate de sodium.

8. Procédé selon l'une quelconque des revendications 6 ou 7, **caractérisé en ce que** la phase aqueuse comprend au moins un agent texturant, de préférence choisi parmi la gomme arabique, le chitosan et le caséinate de sodium.

9. Procédé selon l'une quelconque des revendications 6 à 8, **caractérisé en ce que** la phase aqueuse comprend au moins un agent oxydant, de préférence éthylène-diaminetétraacétique.

10. Procédé selon l'une quelconque des revendications précédentes, **caractérisé en ce que** le milieu hydrophobe comprend une huile choisie parmi les huiles neutres, les huiles essentielles, les huiles polyinsaturées marines ou les huiles polyinsaturées végétales, de préférence le milieu hydrophobe est une huile de poisson.

11. Procédé selon l'une quelconque des revendications précédentes, **caractérisé en ce que** dans les étapes a) et b), le rapport massique entre les lipides amphiphiles et le milieu hydrophobe est compris entre 1/1 et 1/10, et est de préférence 1/3.

12. Procédé selon l'une quelconque des revendications précédentes, comprenant, après l'étape c), une étape d) d'élimination du milieu hydrophobe non encapsulé dans les vésicules.

13. Procédé selon la revendication 12, **caractérisé en ce que** l'étape d) est réalisée par lavage avec une huile hydrophobe différente du milieu hydrophobe utilisé lors des étapes a), b) et c).

14. Procédé selon la revendication 13, dans lequel la phase hydrophobe de l'étape d) est de l'huile de colza.

15. Procédé selon l'une quelconque des revendications précédentes, **caractérisé en ce que** lesdites vésicules sont matricielles.

## Patentansprüche

1. Verfahren zur Herstellung von hydrophoben Vesikeln, die in eine hydrophobe Phase eingliederbar sind, die einen hydrophoben inneren Hohlraum und mindestens eine Doppelschicht umfasst, die aus zwei einzelnen amphiphilen Lipidschichten besteht, die zwischen sich einen hydrophilen Zwischenraum bestimmen, die folgenden Schritte umfassend:
a) Liefern eines hydrophilen Mediums, eines hydrophoben Mediums und von amphiphilen Lipiden,
b) Mischen des hydrophilen Mediums, des hydrophoben Mediums und der amphiphilen Lipide, um Vesikel zu bilden, wobei das Massenverhältnis zwischen dem hydrophilen Medium und den amphiphilen Lipiden zwischen 1/20 und 1/4 liegt,
c) Gewinnen der Vesikel.

2. Verfahren nach Anspruch 1, **dadurch gekennzeichnet, dass** das Massenverhältnis zwischen dem hydrophilen Medium und den amphiphilen Lipiden zwischen 1,5/10 und 2/10 liegt.

3. Verfahren nach einem beliebigen der vorhergehenden Ansprüche,
**dadurch gekennzeichnet, dass** die Mischung bei dem Schritt b) mittels eines dynamischen Mischers, eines statischen Mischers oder Beschallung realisiert wird.

4. Verfahren nach Anspruch 3, **dadurch gekennzeichnet, dass** die Mischung nach Schritt b) durch einen dynamischen Mischer der Art Rotor/Stator realisiert wird.

5. Verfahren nach einem beliebigen der vorhergehenden Ansprüche, **dadurch gekennzeichnet, dass** die amphiphilen Lipide Phospholipide, insbesondere Lecithine, vorzugsweise Sojalecithine sind.

6. Verfahren nach einem beliebigen der vorhergehenden Ansprüche, **dadurch gekennzeichnet, dass** das hydrophile Medium eine wässrige Phase ist.

7. Verfahren nach Anspruch 6, **dadurch gekennzeichnet, dass** die wässrige Phase mindestens einen Emulgator, vorzugsweise ein Maleat von Polyoxyethylensorbitan, Sorbitantrioleat oder Natriumdodecylsulfat, umfasst.

8. Verfahren nach einem beliebigen der Ansprüche 6 oder 7, **dadurch gekennzeichnet, dass** die wässrige Phase mindestens einen Strukturfestiger, vorzugsweise ausgewählt aus Gummi arabicum, Chitosan und Natriumcaseinat, umfasst.

9. Verfahren nach einem beliebigen der Ansprüche 6 bis 8, **dadurch gekennzeichnet, dass** die wässrige Phase mindestens ein Oxidationsmittel, vorzugsweise Ethylendiamintetraessigsäure, umfasst.

10. Verfahren nach einem beliebigen der vorhergehenden Ansprüche, **dadurch gekennzeichnet, dass** das hydrophobe Medium ein Öl umfasst, ausgewählt aus den neutralen Öfen, den ätherischen Ölen, den mehrfach ungesättigten Meerestierölen oder mehrfach ungesättigten
Pflanzenölen, vorzugsweise ist das hydrophobe Medium ein Fischöl.

11. Verfahren nach einem beliebigen der vorhergehenden Ansprüche, **dadurch gekennzeichnet, dass** in den Schritten a) und b) das Massenverhältnis zwischen den amphiphilen Lipiden und dem hydrophoben Medium zwischen 1/1 und 1/10 liegt und vorzugsweise 1/3 ist.

12. Verfahren nach einem beliebigen der vorhergehenden Ansprüche, nach dem Schritt c) einen Schritt d) der Eliminierung des nicht in den Vesikeln gekapselten hydrophoben Mediums umfassend.

13. Verfahren nach Anspruch 12, **dadurch gekennzeichnet, dass** der Schritt d) durch eine Waschung mit einem hydrophoben Öl realisiert wird, das unterschiedlich zu dem hydrophoben Medium ist, das bei den Schritten a), b) und c) verwendet wird.

14. Verfahren nach Anspruch 13, bei dem die hydrophobe Phase des Schritts d) ein Rapsöl ist.

15. Verfahren nach einem beliebigen der vorhergehenden Ansprüche, **dadurch gekennzeichnet, dass** die Vesikel Matrixvesikel sind.

## Claims

1. Process for the preparation of vesicles of hydrophobic character which can be incorporated into a hydrophobic phase, comprising a hydrophobic internal cavity and at least one bilayer made up of two monolayers of amphiphilic lipids defining between them a hydrophilic interstitial space, comprising the stages of:
a) provision of a hydrophilic medium, a hydrophobic medium and amphiphilic lipids,
b) mixing of the hydrophilic medium, the hydrophobic medium and the amphiphilic lipids to form vesicles, the weight ratio between the hydrophilic medium and the amphiphilic lipids being between 1/20 and 1/4,
c) recovery of the vesicles.

2. Process according to claim 1, **characterized in that** the weight ratio between the hydrophilic medium and the amphiphilic lipids is between 1.5/10 and 2/10.

3. Process according to any one of the preceding claims, **characterized in that** the mixing during stage b) is carried out by means of a dynamic mixer, a static mixer or by ultrasound.

4. Process according to claim 3, **characterized in that** the mixing during stage b) is carried out by a dynamic mixer of the rotor/stator type.

5. Process according to any one of the preceding claims, **characterized in that** the amphiphilic lipids are phospholipids, in particular lecithins, preferably soya lecithin.

6. Process according to any one of the preceding claims, **characterized in that** the hydrophilic medium is an aqueous phase.

7. Process according to claim 6, **characterized in that** the aqueous phase comprises at least one emulsifying agent, preferably polyoxyethylene sorbitan maleate, sorbitan trioleate or sodium dodecyl sulfate.

8. Process according to any one of claims 6 or 7, **characterized in that** the aqueous phase comprises at least one texturizing agent, preferably chosen from gum arabic, chitosan and sodium caseinate.

9. Process according to any one of claims 6 to 8, **characterized in that** the aqueous phase comprises at least one oxidizing agent, preferably ethylenediaminetetraacetic acid.

10. Process according to any one of the preceding claims, **characterized in that** the hydrophobic medium comprises an oil chosen from neutral oils, essential oils, polyunsaturated marine oils or polyunsaturated vegetable oils, and preferably the hydrophobic medium is a fish oil.

11. Process according to any one of the preceding claims, **characterized in that** in stages a) and b) the weight ratio between the amphiphilic lipids and the hydrophobic medium is between 1/1 and 1/10, and is preferably 1/3.

12. Process according to any one of the preceding claims, comprising, after stage c), a stage d) of elimination of the hydrophobic medium not encapsulated in the vesicles.

13. Process according to claim 12, **characterized in that** stage d) is carried out by washing with a hydrophobic oil which differs from the hydrophobic medium used during stages a), b) and c).

14. Process according to claim 13, in which the hydrophobic phase of stage d) is rape oil.

15. Process according to any one of the preceding claims, **characterized in that** the said vesicles are in matrix form.
